# EUROPEAN PATENT APPLICATION

(11) **EP 3 058 961 A2**
(43) Date of publication of application: **24.08.2016**
(21) Application number: 14853955.4
(22) Date of filing: 13.10.2014
(51) Int. Cl.: A61L 2/07, A61L 2/02, A61L 2/24, A23L 3/00

(54) **AUTOMATICALLY ROTATING, HIGH-PRESSURE STERILIZER AND PROCESSING METHOD HAVING CONTINUOUS OPERATION STAGES**

(30) Priority: 15.10.2013 CN 201310483188
(71) Applicant: Napasol Asia Ltd., Wanchai Hong Kong (CN)
(72) Inventor: WAENY, Ursula, CH-3920 Zermatt (CH)
(74) Representative: Gasquet, Denis
(86) International application number: PCT/IB2014/002083
(87) International publication number: WO 2015/056071

(57) **Abstract**

The present invention provides an automatic rotary autoclave 10, a vessel 110 of which has a longitudinal axis 151 inclined to a horizontal rotation axis 150, wherein the treatment chamber 114 of the vessel 110 has a smooth and protrusion-free inner surface 114a or a member for mechanically blending product 100, and wherein the vessel 110 at least has: means 140 ∼ 144 for supporting and rotating the vessel, a first coupling 120, means 121 ∼ 123 for temperature controlling, means 131 ∼ 134 for supplying the treatment chamber 114, sensor means 145, 150, and a PLC type electronic controller 163. The present invention also provides a corresponding treatment process. With the present invention, relatively high quality can be achieved with relatively low operation cost, and relatively high yield can also be achieved.

## Description

### TECHNICAL FIELD

The present invention belongs to disinfection and sterilization field. An object of the present invention is to provide an automatic rotary autoclave for carrying out combined pasteurization and blending operation on organic materials in powder form or as particulates under saturated steam conditions; a further object of the invention is to provide a treatment procedure for said materials by the means of said rotary autoclave.

### BACKGROUND

The innovation is especially, but not exclusively, suitable for its application in the area of the industrial production of food products, finding also applications in the treatment of non-food products, for example in the sector of the cosmetic and pharmaceutical industry; the invention is particularly efficient for the treatment of bulk products in powder form or as particulates with a low moisture content, such as for example, but not limited to cereals, dried fruit, coffee or spices. Moreover, the invention is capable of combining treatments including blending, pasteurization or sterilization of any organic bulk material in powder form or as particulates with low moisture content.

Among the well-known and conventional treatments for the conservation of food products, there are physical methods using heat at a specific temperature level and during a defined period of time, to eliminate or inhibit the growth of microorganisms which generally will contaminate organic materials, such as, in the case of food products, bacteria, mold, yeast, and viruses. In particular, it is known that among the factors, the presence of a specific temperature and moisture is a reason of the development of microorganisms to a great extent; in the specific case of low moisture food products, such as dried fruit or cereals, depending on their thermal resistance, the treatments which are mostly used for the reduction of the bacterial load utilize the step of heating the product to a high temperature level.

Among the well-known solutions which are generally used to reduce such bacterial load, the pasteurization and the sterilization are specifically mentioned. The procedure called pasteurization generally provides temperatures in the range between 60°C and 100°C during a time period between 15 seconds and 30 minutes, depending on the defined cycle and on the applied temperature, the time period will be shorter at higher temperature levels; different pasteurization methods substantially destroy pathogenic bacteria all by denaturing the enzymes. The process conventionally called sterilization, on the other hand, is used to destroy more resistant microorganisms and spores, providing temperatures in the range between 100°C and 150°C during a time period between a few seconds and more than 20 minutes, the time period will be shorter at higher temperature levels.

In principle, said pasteurization and sterilization treatments are technologically similar, using a system with a closed treatment chamber, which allows the use of heat in a homogeneous and controlled way. In both cases, the treatment chamber has to be hermetically closed and it has to be designed to work at a pressure range different from atmospheric pressure; such a machine configuration for pasteurization or sterilization is conventionally called autoclave.

Conventionally, the term "pasteurization" is used for any type of thermal treatment of organic products which aims for the extension of the shelf life of the product, destroying or inhibiting the development of said microorganisms as well as insects or other types of pests. In the present description, the generic term of pasteurization will be used in its broadest meaning, thus including the thermal sterilization treatment as conventionally understood.

Among the processes generally used in the food industry, there is also a blending process, the particular objective of which is to mix different organic bulk products that are in powder form or as particulates, have a low moisture content, and have different characters from each other or have the same characters but of different shapes or particle sizes; as an example, such a procedure is generally used to achieve homogeneous mixing of cereals, dried fruit or spice blends, and it is also used in the production of fodder, and in the pharmaceutical and cosmetic industries. Most blending systems rely on the mechanical action of a paddle, screw or scraper to mix the ingredients; other blenders of dry foods that do not use this mechanical action are based on the physical properties of gravity and rotation to obtain a homogenous mixing of ingredients.

Furthermore, it is also known that said pasteurization and said blending are often performed both on the same products before packaging, not simultaneously, but successively in different machines; such machines, in fact, are based on different technologies: a pasteurizer provides thermal treatment in a static, hermetically closed chamber, resulting in the reduction of the microbial load of the product; a blender, instead, is substantially a machine which mixes different products, generally functions through the rotation of the whole vessel which contains the products to be blended, or through rotating devices inside the vessel, such as paddles or spirals, sometimes mounted in series.

It is also known that conventional solutions, although widespread and standardized, present significant and unresolved production problems. In the case of the pasteurization or sterilization, such problems mainly concern the homogeneity of the treatment throughout the load and the optimized control of the entire cycle and of the respective process parameters, as well as the agglomeration of the product; in the case of the blending, on the other hand, the problems are mainly related to the mechanical damage resulting from agitating the product during the blending cycle. In addition to the above mentioned problems, it is also widely known that, in addition to the general problem of cleaning of all the surfaces which are in contact with the product, both of said processing technologies present the serious risk of contamination during the loading and unloading phase, particularly in the case of closed machines and when changing the products to be processed.

In the food industry, the pasteurization or sterilization process is often performed at the end of the complete production process and immediately before the packaging of the product, with the objective to reduce as much as possible the risks related to food safety and to extend the shelf life of the product. This treatment is otherwise called a kill step. Such a treatment can be performed advantageously by using steam; in order to achieve an optimized efficiency and to minimize the impact on the product characteristics, the use of dry, saturated steam is preferable, as it has a higher latent thermal energy compared to wet steam or superheated steam, and is therefore more efficient to reduce the microbial load and also to reduce the problem of product agglomerations which is known to be frequently happened in the case of bulk products in large treatment chambers and in the case of mixed products.

### Prior art

The object of the present invention is therefore intended to seek for an optimized solution for an autoclave for the combined pasteurization and blending treatment of organic materials in powder form or as particulates, with specific reference to food products in bulk; a research of prior art was carried out through patent literature, which is manifested in some relevant documents:
D1: WO2009003546 (Perren et al.)
D2: GB1243823 (Loedige et al.)
D3: GB2249706 (Lucas)
D4: FR2680637 (Antonini et al.)
D5: WO9843682 (Forberg)
D6: EP 1508364 (Taniguchi)
D7: JP2005334854 (Nakano)
D8: EP1205112 (Dreano et al.)
D9: GB1445942 (Nestle)
D10: GB2302258 (Fantozzi et al.)
D11: EP1450868 (Howe et al.)

D1 described a pasteurization or sterilization process for food products in particulate form, which involves preheating the product in a separate vertical vessel to a temperature close to the treatment temperature, performing the pasteurization with steam and removing the water on the particle surface by means of a vacuum pump; the machine for this treatment is a vertical vessel with an internal helicoidally shaped agitator. The vessel has a double jacket with cavity for its heating and cooling, and it is also fitted with a plurality of accessory systems capable of achieving vacuum, increasing the internal pressure, and introducing steam. D2 proposed a steam sterilization process in a closed, horizontal cylindrical chamber, for powder or small particle materials, which also involves a continuous agitation or mixing of the product during the different phases of the treatment; D3, instead, described a machine for the steam sterilization of animal feed, with a closed, horizontal cylindrically shaped chamber which is equipped with gates for loading and unloading the product on opposite sides thereof, and also equipped with a internal conveying system of a helicoidally shaped screw type on its longitudinal axes, which allows a continuous treatment to a certain extent.

D4 proposed a method of pasteurization and sterilization for agro-food products in bulk by means of a machine which includes an open chamber allowing a thermal treatment to be done in a horizontal, elongated vessel, wherein on the feeding side the material is continuously introduced and moved by a vertically and horizontally vibrating table; D5 described a method for steam sterilizing materials in granulate form or small particles, the particles are agitated in a first chamber by two parallel rotors with horizontal shafts and are exposed to steam, then transferred by gravity to a chamber below where they are moved by a gear worm. D6 proposed a steam agitator-pasteurizer wherein the agitating device is centrally mounted in a chamber along a longitudinal axis; said chamber is fitted with a surface heating system by liquid and it is also fitted with a system for filtering the material at the exit of the chamber.

D7 proposed a horizontally mounted mixing tank for food products which is equipped with two parallel paddle agitators that are arranged inside the tank and articulated at side walls, the tank can be emptied by tilting by means of rotating around said axis; D8, instead, described a cylindrical vessel with a hermetically closed door for processing foods in brine, capable of rotating on its own axis and also at the time of being tilted, and internally equipped with mixing blades, which span radially over the whole length, up to its base.

### Closest prior art to the invention

D9 proposes a sterilization process of solid materials of small particles in a rotating vessel, which moves the product during a thermal treatment through steam, under sterile conditions. D10 describes a system for reducing the bacterial load in organic materials of the herbs or spices type by means of a rotating and conditioning tank, the tank is shaped like a double truncated cone symmetrically opposed and having the tips at the opposite ends; they are opposite in such a way as to allow separately loading on the top and unloading at the bottom by gravity, being also pivotable to a side about the horizontal centerline to be rotated during the treatment; in particular, said vessel has a double jacket with cavity for its heating or cooling, is also provided with a plurality of accessory systems allowing to obtain vacuum, increase the internal pressure, and recuperate aromas during processing.

D11 described a method and its corresponding equipment for reducing the bacterial load in organic materials by means of a continuous flow of saturated steam inside a hermetically closed chamber, and wherein the temperature and the pressure are controlled and correlated according to the tables of saturated steam, also known in physics as Saturated Steam Tables. The treatment process includes the following phases: a) introducing the material into the chamber, b) selecting the desired temperature according to said material, c) selecting the correlated pressure, d) creating vacuum, e) introducing steam until the pressure reaches the selected value, and f) maintaining the pressure and temperature values during the correct treatment time period. To speed up the process, in an alternative configuration, there is a preheating phase.

### Disadvantages

Indeed, is has been known that the major part of the described solutions present some disadvantages or at least they have limitations.

In D1, difficulties in the cleaning of the machine are revealed because of the difficulties in accessibility and also problems of removing the product from the pasteurization vessel, the opening is of a small diameter; furthermore, the machine will allow stirring the product in case it remains stuck inside the vessel, which causes mechanical damage to the product itself. D2 neither provides the use of saturated steam nor the preheating of the product, the vessel is equipped with a double jacket only insulated but not temperature controlled; a mixing mechanism is integrated which is difficult to clean and can damage delicate products; furthermore, there is no drying capability integrated, which makes it necessary to perform such function in a second external vessel.

D3 does not involve the use of saturated steam, but performs the sterilization of the product with wet steam which has the particularity to cook the product and to gelatinize its starch content, it reveals that such a treatment is different from the present invention because it results in an excessive change of the product and also will damage the product due to the mechanical action of the screw; also in D4 and D5, the product is moved by a screw which can cause damages to delicate products and results in difficulty in cleaning the vessel; it is also obvious that the moving system cannot be used as a blender and that the product needs a drying phase subsequent to the treatment. D6 does not work in saturated steam conditions and it is also obvious that the function of the agitator improves the fluidity of the product to be treated, but may result in damage to delicate products and is difficult to clean. Finally, it is clear that D7 and D8 provide a correct blending of the product but no pasteurization.

D9 is a cooking system which does not involve saturated steam and is not suitable for low moisture food products; furthermore, it results in mechanical damages of delicate products and is difficult to clean. D10 performs the thermal treatment of the product by means of convectively heating and cooling the double jacket only; there is no direct contact of the steam with the product and the efficiency of the treatment is limited. D11 is a static process without blending under saturated steam conditions, with a very long treatment time in order to allow the steam to penetrate into the product and with the possibility that this is achieved in a non-homogeneous way due to the high humidity without the possibility of drying; such a system favors the formation of lumps of the material and is obviously less efficient for the industrial treatment of food products with a low moisture content and has no blending capacity. While it is possible with the present invention where a high level of quality and capacity is required.

With reference to the objectives of the present invention, the following disadvantages and limitations found in the known solutions are particularly noticeable: high risk of cross-contamination, limited efficiency and long duration of the treatment, difficulty in control of the process parameters and, especially in the cases that saturated steam and vacuum drying are not used, change of the product quality following the treatment and particularly high moisture absorption, impossibility to get a perfectly blended product without agglomerates at the end of the cycle for packaging, difficulty in integrating different functions in one compact and automated installation. Generally, it was also found that the blending and agitating systems enable the product to be fluidized and improve the treatment using paddles or screws which causes damage to delicate products of the type of bulk food products in powder form or particulates with a low moisture content, such as and not limited to, the cereal mixes, flakes or blends of dehydrated vegetables due to mechanical movement. Furthermore, there will be some large and complex surfaces to be cleaned with numerous gaps where the dirt is difficult to remove, especially when there are mobile elements such as paddles and spirals of the mixers.

From these introductory remarks we can conclude that it is very important to identify alternative solutions, which are more effective.

### SUMMARY

This and other scopes are achieved by the present innovation according to the features in the included claims, which solves the explained problems by means of a rotary autoclave 10 using saturated steam in an inclined vessel 110, the rotary autoclave 10 is used for the pasteurization and sterilization of granular products 100 with a low moisture content, combines with a blending function to optimize the treatment and delivers a product which is ready for packaging. A particular, automated treatment process is used, which has at least the following operational sequential phases: a loading phase A, a tempering phase B, a pre-vacuum phase C, a pasteurization phase D, a post-vacuum phase E, a discharging phase F, and an unloading phase G.

### Scopes

In this way, through the appreciable creative contribution, the effect of which constitutes an immediate technical progress, several advantages are achieved.

A first scope consists in achieving an autoclave type machine for organic bulk materials in powder or particulate form, which combines at the same time the functions of blending and pasteurization - sterilization, said autoclave is of a rotary type and is also optimized to use saturated steam according to a particular treatment process; particularly, said combination of functions is achieved in such a way that a result of an extremely homogeneous and efficient treatment on the whole load is obtained, without agglomerates. At the same time such a rotary autoclave allows executing the treatment cycles which conventionally are called pasteurization, or cycles which conventionally are called sterilization.

A second scope consists in achieving a rotary autoclave using saturated steam which allows achieving a perfectly blended product at the end of the cycle for packaging, also in case of different materials and/or different types and particle sizes, without agglomerates; this advantage is particularly achieved for bulk products with low moisture content, including those in the form of powder.

A third scope consists in achieving a rotary autoclave using saturated steam which reduces the risk of cross-contamination and allows a quick and efficient cleaning of the surfaces which are in contact with the organic, treated material, facilitating the removal of dirt; such characteristics, in particular, refer to the potential contamination of organic material which will be introduced in the subsequent treatment cycle, also relate to the known problem of allergens as it happens frequently when changing products.

A fourth scope, directly related with the preceding scopes, consists in achieving a particular integrated system for injecting steam and vacuum extraction, the system uses the same channel for conveying both alternating flows, the channel is fixed and concentric to the axis of rotation of the vessel and equipped with a self-cleaning filter; in particular, the system is optimized in such a way as to allow to reduce the risk of contamination, and to handle the steam injection and the vacuum extraction in an integrated way, to avoid impacting the effectiveness of the treatment in spite of the complex and mutually interdependent effects.

A fifth scope consists in achieving a combined blender and pasteurizer-sterilizer in such a way to optimize the treatments and to allow for a significant saving on floor space, weight, building and environmental cost, time, and the overall processing cost; the two functions, in fact, take place at the same time in the same vessel integrated in a compact way and sharing various accessory systems and components.

A sixth scope consists in achieving a rotary autoclave for the pasteurization-sterilization, which allows an integrated and optimized control of the complete treatment cycle.

Another scope consists in achieving an autoclave type machine for organic bulk materials with low moisture content, in powder or particulate form, which is extremely versatile and customizable regarding its use according to specific treatment cycles, executing the thermal treatment and the blending of the products at the same time.

A further scope is intended to achieve a particular manufacturing process which is adapted to pasteurize - sterilize mixtures of granular products with low moisture content that cannot be treated with currently known systems in an effective way, allows high yield and low costs. In particular, different from all prior art where paddles or screws are used to mix and fluidize the products, the present invention has an effective blending capacity without breaking very fragile product particles. Therefore, it is possible to use this invention for the blending and pasteurization - sterilization of relatively fragile products, like e.g. cereal mixes, flakes, or dehydrated vegetable mixtures.

According to an aspect of the present invention, an automatic rotary autoclave 10 is provided, which is used under saturated steam condition for pasteurization - sterilization of a low moisture product 100 in particulate form, and for blending and mixing, the automatic rotary autoclave 10 includes a hermetically closed cylindrical vessel 110 having a complete treatment chamber 114 and also of double jacket 111, 112 type, the double jacket define a treatment cavity 113, and the vessel 110 is moved by an electrical motor, the autoclave is characterized in that the vessel 110 has a longitudinal axis 151 inclined to a horizontal rotation axis 150, wherein the treatment chamber 114 of the vessel 110 has a inner surface 114a which is smooth and has no protrusions or a member for mechanically blending the product 100, and wherein the vessel 110 at least has:
means for supporting and rotating the vessel;
means for controlling temperature of said treatment cavity 113 of the double jacket through a first coupling 120 as a rotary coupling positioned to be connected with said horizontal rotation axis 150;
means for supplying said treatment chamber 114 alternately with steam injection or steam extraction through a second coupling 130 as a fixed coupling, wherein on the opposite sides of the means for controlling temperature with respect to said treatment chamber 113, the vessel also has a system for filtering and diffusing inside the treatment chamber 114, said second coupling 130 as the fixed coupling is positioned to be connected with said horizontal rotation axis 150, and the treatment chamber 114 is connected with the second coupling 130 as the fixed coupling and is of a self-cleaning type;
sensor means for detecting rotation and position of the vessel 110 with respect to the horizontal rotation axis;
a PLC type electronic controller 163 which is connected in an integrated way at least to said means for controlling temperature, said means for supplying, and said means for supporting and rotating, and at least connected to temperature and pressure sensing means and a rotation sensing means 145 in order to monitor and automatically adjust values of pressure and temperature in the treatment chamber 114 so as to achieve and maintain the correct treatment conditions of the product 100 even during the rotation of the vessel 110 in the presence of the saturated steam, said electronic controller 163 being adapted to manage automatically the operation of the automatic rotating autoclave 10 and the whole treatment process.

It is preferred that said vessel 110 is rotated on the horizontal rotation axis 150 in such a way that the vessel 110 can reach its loading position at which the opening 115 is located at the highest point of the vessel or its unloading position at which the opening 115 is located at the lowest point of the vessel, said opening 115 is also equipped with a valve 116; said vessel 110 is used as an agitator-blender combining low speed rotation with the gravitational mass of the product 100; and said means for controlling the temperature of the treatment cavity 113 comprise an external heater 121 with an agitator for hot fluid which is introduced through the first coupling 120 as a rotary coupling, and the first coupling 120, as a rotary coupling, is of the dual channel concentric type, and wherein said means for supporting the treatment chamber 114 comprise at least one vacuum pump, a steam generator, said second coupling 130 as a fixed coupling is of a single-channel and bi-directional type and also provides a mechanical seal 136 of the vessel 110 with a closure member in such a way as to allow the vessel 110 to be rotated simultaneously and independently during the vacuum extraction and during the steam injection, and wherein said filtration and diffusion system is achieved by a filter of the wedge wire screen type, and wherein said temperature and pressure sensing means combines the sensors for detecting the pressure and temperature of the hot fluid in the treatment cavity 113 of the double jacket, and wherein said electronic controller 163 is electronically integrated with said valve 116, said means for controlling temperature, said means for supplying, said means for rotating, said temperature and pressure sensing means in such a way as to automatically manage the operation of the vessel according to a automatic treatment program which is selected when the cycle is initiated according to the product 100 to be treated and the treatment to be performed; said automatic treatment program enables to instruct said controller 163 with the correct operating parameters in such a way that the correct saturated steam conditions are achieved and maintained in the whole treatment chamber 114 during its rotation.

It is preferred that said vessel 110 is made of food grade stainless steel metal having an inner capacity of the treatment chamber 114 in the range between 200 and 30000 liters; and wherein said vessel 110 has said longitudinal axis 151 inclined with respect to the horizontal rotation axis 150 of the automatic rotary autoclave 10 by an angle in the range between 30° and 40°; and wherein said speed of slow rotation is in the range between 2 rpm and 15rpm.

According to another aspect of the present invention, a treatment process having continuous operation phases by means of the above mentioned automatic rotary autoclave is provided, said treatment process at least provides, in a logic sequence, the following phases A ∼ G:
Phase A) loading phase, including at least: sub-phase A1) initiating a predefined program that is from the electronic controller and standard recorded; sub-phase A2): entering a loading position through the opening of the valve; sub-phase A3) loading the product in the vessel; sub-phase A4) cleaning the valve by air suction, retracting an external feeding hopper and closing the valve.
Phase B) tempering phase, allowing the preheating of the material, including at least: sub-phase B1) heating the treatment cavity by introducing hot fluid, steam or water;
Phase C) pre-vacuum phase, increasing the temperature of the treatment chamber in a short time up to a predetermined pressure level by means of a rapid vacuum extraction followed by a rapid steam injection, and wherein the vacuum and the steam pass through the same single bi-directional and self-cleaning filter, and wherein the phase C) is performed one time or in repetitive manner, always beginning with the vacuum and terminating at steam injection, wherein said phase C includes at least: sub-phase C1): achieving a rapid vacuum; sub-phase C2): rapidly injecting steam; sub-phase CR): slowly rotating the vessel 110 during the sub-phases C1 and C2.
Phase D) pasteurization phase, wherein to maintain the correct pasteurization or sterilization temperature under saturated steam conditions, adjustment is provided by the controlled injection of steam into the chamber 114 and the temperature control of the treatment cavity 113, said phase D includes at least: sub-phase D1): injecting steam into the treatment chamber 114, wherein pressure values on the product 100 are achieved corresponding to the pre-selected temperature, which is in the range between 60°C and 121°C; sub-phase D2) maintaining the pressure and temperature levels during the whole defined duration of the treatment; sub-phase DR) slowly rotating the vessel 110 during the sub-phases D1 and D2.
Phase E) post-vacuum phase, cooling and drying the material, including at least: sub-phase E1) extraction of vacuum; sub-phase E2) eventually, in the pulse type treatments, releasing air through a valve with a sterile air filter; sub-phase ER) slowly rotating the vessel 110 during sub-phase E1 and the final sub-phase E2.
Phase F) venting phase, allowing the treatment chamber to return to atmospheric pressure for discharging, including at least: sub-phase F1) opening the valve to release sterile air into the treatment chamber until the ambient pressure is reached; sub-phase F2) stand-by and waiting for manual activation; sub-phase FR) slowly rotating the vessel 110 during sub-phase F1.
Phase G) unloading phase, manually activating, including at least: sub-phase G1) moving the vessel 110 to the unloading position; sub-phase G2) activating the discharge connection; sub-phase G3) opening the valve and unloading the product; sub-phase G4) cleaning the valve, and making the automatic rotary autoclave 10 ready for the subsequent processing cycle.

It is preferred that, in the sub phase D2), the pressure and the temperature level during the whole defined duration of the treatment is maintained for the period in the range between 1 min. and 20 min.

It is preferred that, the phase B) includes sub-phase BR) of slowly rotating the vessel 110 during the sub-phase B1.

With the present invention, high quality is achieved with relative low operation cost, and high yield can also be achieved.

These and other advantages will be apparent from the following detail description of some preferred embodiments with reference to the attached drawings, of which the details to be implemented therein should be deemed as illustrative, rather than limitative.

### BRIEF DESCRIPTION OF DRAWINGS

These and other advantages will appear from the detailed description of some non-limitative embodiments with the aid of the following drawings.
Figure 1 is an orthogonal view of the autoclave object of the present invention in a vertical section, wherein a dashed line and letter A or B indicate enlarged details regarding the single enlarged detail views as shown in Figs. 2 and 3;
Figure 2 is an enlarged view of the detail A as shown in Fig. 1, which shows a rotary coupling with a double channel for the thermal control of a cavity of a double jacket;
Figure 3 is an enlarged view of the detail B as shown in Fig. 1, which shows a single-channel fixed coupling for supplying a treatment chamber with the steam injection and the extraction of a vacuum, this view also shows the detail C showing the enlarged section of a filter;
Figure 4 is a simplified schematic of the treatment process, which is the object of the present invention.
Figure 5 is a double diagram which, as an example, shows in a graphic way the relationship between the temperature values and the pressure during the phases B-F of the treatment process as shown in Fig. 4, wherein the parameters are particularly optimized for the pasteurization of a blend of cereals containing oatmeal, raisins, and pieces of walnuts.
Figure 6 is a report showing the result achieved through the pasteurization treatment as shown in Fig. 5, describing a series of nine repeated test cycles under the same condition, and wherein the reduction of the bacterial load in the product is expressed at a logarithmic scale.

In the drawings:
10 - autoclave
100 - product
110 - vessel
111 - first jacket
112- second jacket
113 - treatment cavity
114 - treatment chamber
114a - surface
115 - opening
116 - valve
117 - manhole
118 - viewing window
119 - support leg
120 - first coupling
121 - external heater
122 - first conditioning fluid
123 - second conditioning fluid
124 - flange
125 - fixed support
126 - external pipe
127 - pneumatic coupling
130 - second coupling
131 - first vacuum pump
132 - second vacuum pump
133 - first steam generator
134 - second steam generator
135 - connection pipe
136 - mechanical seal
137 - first filtration system
138 - second filtration system
140 - motor
141 - chain system
142 - gear
143 - side support leg
144 - rotary support
145 - fixed sensor for positioning
146 - rotary locator
147- collector
150 - horizontal rotation axis
151 - longitudinal axis
160 - chamber temperature probe
161 - product temperature probe
162 - pressure transducer
163 - electronic controller
170 - external feeding hopper
110a - showing the vessel at the loading position
110b - showing the vessel at the unloading position
115a - showing the opening at the loading position
115b - showing the opening at the unloading position
151a - the longitudinal axis at the loading position
151b - the longitudinal axis at the unloading position

### DETAILED DESCRIPTION

Hereinafter, the present invention will be described in detail in connection with the particular embodiment thereof. The embodiments below will assist the person skilled in the art to understand the present invention, but will not limit the present invention in any way. It is to be noted that several variations and modifications can be made by the person skilled in the art without departing from the concept of the present invention. These variation and modification should fall within the protection scope of the present invention.

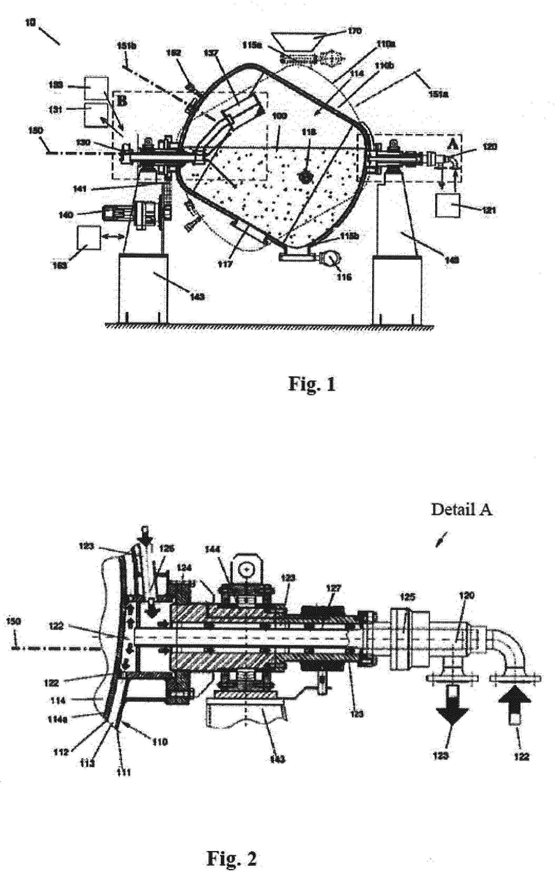

Also with reference to the drawings (Figs. 1 - 6), the invention describes a autoclave type machine using saturated steam, capable of achieving the scopes described above, in which, particularly, the optimization of the industrial processes of pasteurization - sterilization and the blending, also in combination with homogenizing the treatment and eliminating the formation of agglomerates, significantly reduces the risk of cross-contamination of the treated materials, facilitates cleaning and has the capability of treating fragile materials without damaging them. This is made possible by means of an innovative apparatus of the type of a rotary autoclave 10 using saturated steam; such equipment is hereinafter referred to as an autoclave for descriptive simplicity, although advantageously it comprises a plurality of devices and functions that make it more complex, compared to a conventional autoclave. Additionally, in order to achieve these purposes by using said rotary autoclave 10, a particular automatic treatment process has to be used, which consists of a sequence of operative phases (phases A - G) and sub-phases, as described below.

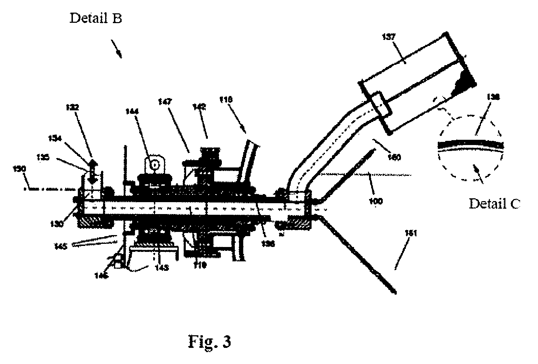

Said rotary autoclave10 has a treatment chamber 114 for granular product 100 inside a cylindrical inclined vessel 110 which can be hermetically closed, controlled and rotated, allowing creating various conditions of pressure and temperature inside with respect to the external ambient conditions. In the preferred embodiment shown in Fig. 1 to Fig. 3, in particular, said inclined vessel 110 has a double jacket (including a first jacket 111 and a second jacket 112) with a treatment cavity 113 therebetween, the treatment cavity 113 is temperature controlled with fluid, for example hot water or steam, and is fixed on an inclined longitudinal shaft 151 a, 151b in such a way as to permit a balanced rotation around a horizontal rotation axis 150, said inclined vessel 110 is supported laterally at a first coupling 120 and a second coupling 130, to control, through the first coupling 120 and the second coupling 130, the fluid in the treatment cavity 113 of the double jacket (Fig. 2) and the treatment chamber 114 (Fig. 3) to be conducted through the horizontal rotation axis 150 in a centralized way, to allow the inclined vessel 110 to be freely and independently rotated during the treatment cycle. More specifically, such rotation of the inclined vessel 110 around the horizontal rotation axis 150 is occurred in parallel with the temperature control of the treatment cavity 113 of the double jacket as well as the extraction of the vacuum or the injection of steam into the treatment chamber 114.

An inner surface of said inclined vessel 110 is smooth and free from protuberances in order to facilitate the cleaning. It is preferable to carry out mechanical polishing on all the inner surfaces, for example, as viewed from the inner side of the chamber 114, the surface 114a avoids the treated product 100 sticking to these surfaces to a certain extent, and facilitates the total unloading of the vessel.

In order to facilitate the blending and the fluidization during the treatment, said inclined vessel 110 has its longitudinal axis 151 inclined at an angle between 30° and 40° with respect to the horizontal rotation axis 150, so as to combine the gravitational mass of the granular product 100 with a balanced rotation of slow speed, for example between 2 and 15 rpm. This inclined configuration of the longitudinal axis 151 also facilitates the loading and unloading operation through an opening 115 with a valve 116 which is arranged at the highest point 110a, 115a, 151a in the loading position, or at the lowest point 110b, 115b, 151b in the unloading position, the operation is induced by rotating 180° about the horizontal axis 150 to automatically change from one position to another position. Said inclined vessel 110 is preferably made of stainless steel with a thickness corresponding to the magnitude of the action of the internal pressure conditions, wherein the treatment chamber 114 has an internal capacity in a range between 200 and 30,000 liters, and the pressure is in a range between -1.0 barg and +1.5 barg. Additionally, there are a viewing window 118 and a manhole 117 for allowing access to the interior of the autoclave for maintenance and cleaning.

In addition to said inclined vessel 110 in the preferred embodiment, the rotary autoclave 10 according to the present invention includes at least the following elements:
- lateral supporting legs 143, on top of which rotary supports 144 constructed by conventional supports are arranged to allow the inclined vessel 110 to be rotated about the horizontal rotation axis 150;
- a system for rotating the inclined vessel 110 to the horizontal rotation axis 150, including a motor 140 of an electric motor type with an inverter to allow its rotation speed to be adjusted between 0 rpm and 15 rpm, and also including transmission devices such as, for example, a chain system 141 and a gear 142;
- a system for controlling temperature of the treatment cavity 113 (Figs. 1 and 2) of the double jacket, which enables the treatment chamber 114 to operate under saturated steam conditions in quick and controlled way, i.e. in the most optimal conditions for the maximum effectiveness of pasteurization, and includes at least an external heat generator equipped with a circulator 121 with a first conditioning fluid 122 and a second conditioning fluid 123 (e.g. steam or water) which are injected through the rotating couplings (including a first coupling 120 and a second coupling 130) with double channels. For example, black steam produced by a quick reaction steam generator can advantageously be used as a temperature control fluid.
- a rotary coupling having concentric (Fig. 2) double channels 120 in the type named Johnson Fluiten, allowing the incoming first conditioning fluid 122 to be released into the treatment cavity 113, and the incoming second conditioning fluid 123 to heat an inner wall of the second jacket 112 in direct contact with the granular product 100, and allowing to draw it out and back into the rotary coupling through an external pipe 126;
- means (the second coupling 130, the first vacuum pump 131, the second vacuum pump 132, the first steam generator 133, the second steam generator 134, the first filtration system 137) for alternatively supplying said treatment chamber 114 with steam or extracting said treatment chamber 114 under vacuum through a second coupling 130 as a fixed coupling, also consisted of the first filtration system 137, the second filtration system 138, the first vacuum pump 131, the second vacuum pump 132, the first steam generator 133, and the second steam generator 134,
- a second coupling 130 used for the treatment chamber 114 (Fig. 1, Fig. 3) and working as a joint service device, disposed symmetrically on the horizontal rotation axis 150 with respect to the first coupling 120 as a rotary coupling, being of single-channel and bi-directional type for the injecting steam or sterile air, or for the extraction of vacuum; wherein said second coupling 130 is fixed and integrated with the support structure, and terminated inside the treatment chamber 114 having the first filtration system 137 and the second filtration system 138, and provides also a closure element to a mechanical seal 136, which allows the tank to be rotated under a positive or negative pressure;
- a self-cleaning type filtration and diffusion system inside the treatment chamber 114, which includes particular filters in the type of the first filtration system 137 referred to as a first wedge-shaped wire screen and the second filtration system 138 referred to as a second wedge-shaped wire screen, to allow the air or the steam leaving the treatment chamber 114 to be effectively filtered during the vacuum extraction phase and also to allow the steam and sterile air to pass in such a way that effective cleaning of the filter is performed simultaneously. Such filtration system prevents the granular product 100 from moving into the various auxiliary components, and also maintains itself clean and ready for the next phase with maximum efficiency and safety, without interfering with the rotation of the inclined vessel 110;
- a first external vacuum pump 131 and a second vacuum pump 132, which are preferably in the type referred to as single stage or liquid ring vacuum pump, suitable to establish vacuum in the treatment chamber 114 in a short time with high pressure so as to prepare for the granular product 100 to receive the steam quickly and homogenously;
- a first steam generator 133 and a second steam generator 134, made of stainless steel, and configured for injecting clean steam into the treatment chamber 114 through a connection pipe 135, wherein said second coupling 130 and said first filtration system 137 are for the purpose of treating the granular product 100 in saturated steam conditions. Optionally, it is also possible to use a carbon steel steam generator for producing steam with a steam converter which is made of stainless steel and is used to convert black steam into clean steam preferably made of osmotic water, to heat the treatment cavity of the double jacket; this clean steam is used as process steam in direct contact with the products;
- sensors for controlling the rotation of the inclined vessel 110, also for controlling fixed sensors 145 for positioning at the positions for loading and unloading the vessel, a rotation locator 146 arranged around the horizontal rotation axis 150, and a collector 147 for data transmission for example having electric rotary couplings connected to a pressure transducer 162 and to an end switch of a loading - unloading valve 116;
- a pneumatic coupling 127, also for the actuation of the loading and unloading;
- includes, but not shown in the figures: an external filter which is of the sterile type and equipped with a valve for injecting filtered ambient air into the chamber, an electric valve in the type of a proportional valve to precisely control the injection of the steam into the autoclave, a heat exchanger connected so as to supply cooling fluid to the vacuum pump for optimal performance, a heat exchanger for cooling and condensing the steam and humid hot air from the autoclave which is equipped with a condensate evacuation system and a condensate recovery system to reduce water consumption during the cycle, an external air compressor in the type called oil-free screw compressor which supplies the filtered, compressed air into a tank preferably equipped with an UV-light to prevent any microbial build-up, and conventional systems for the safety of pressure vessels;
- a PLC (Programmable Logic Controller) type electronic controller 163, for rotating the autoclave 10 and for automatically controlling the whole procedure of automatic treatment process, for example the controller called Siemens Simatic S7 - 300 - CPU314C, including a card for connecting to an Ethernet network and a router for connecting to the internet, for the purpose of remote assistance and trouble shooting, and also being connected to a PC with a touch screen on which the programs for treatment process and remote assistance, such as customizable software called Simatic WinCC RT are installed. Said PLC can be mounted in the electrical control board.

Then, said PLC type electronic controller 163 manages by using modules of conventional type where software can be customized and parameterized with the variation of said rotary autoclave 10 and of the automatic treatment method, is specific to the granular product to be treated 100 or to the blend of products, and is dependent on the particular treatment to be performed. More specifically, the invention allows said module to be customized and parameterized in such a way that the entire treatment cycle can be initiated manually by choosing a specific treatment program comprising a complete set of instructions and parameters characterizing the particular procedure to be performed by means of the touch screen connected to the PLC by an operator in a quick and intuitive way. In order to allow properly managing the industrial production and quality, said program, when being executed, will then be combined with a certain number of standardized routine information, for example, but not limited to, the date, the lot number, the name of the processed product, the name of the operator, the sequential number of the cycle.

Said PLC type electronic controller 163 allows all stages of the cycle to be precisely and automatically controlled, said software is parameterized according to the critical variables of the process, which are at least the temperature of the product, the temperature of the chamber, the temperature of the double jacket, the pressure, the duration of pasteurization phase and the speed of rotation, all monitored and controlled by said sensors in a way so as to quickly adjust the process according to the measured values. At the end of the cycle, it is then possible to automatically obtain all the values recorded during the execution of the process, including eventual deviations from the set values.

It is noted that said rotary autoclave 10 integrally incorporates a plurality of functions in a complex manner, also includes fixed and rotating elements which are combined in the first coupling 120 as a rotary coupling with the rotary chamber and the second coupling 130 as a fixed supplying coupling, wherein both coupling is centralized on the horizontal rotation axis150. In particular, due to the specially designed single first filtration system 137 which is fixed and shaped in a way to adjust the inclination and balance of the rotating vessel 110, it allows its free rotation, independently from the temperature control on the injection of steam into the first vacuum pump 131 and the second vacuum pump 132, the vacuum extraction of first steam generator 133 and the second steam generator 134 and the treatment cavity of the double jacket, and allows the procedures determined and claimed according to each of the separate process routines being executed separately or jointly.

The rotary autoclave 10 constructed as described above allows an optimized pasteurization treatment to be performed preferably in a temperature range between 60°C and 99°C and at a relatively low pasteurization pressure, but also allows sterilization to be performed at a temperature range between 100°C and 121°C, at a relatively high pressure; said treatments, in combination with a perfect blending performed at a rotation speed between 2 and 15 rpm, also provides a blended, pasteurized finished product 100 ready for packaging.

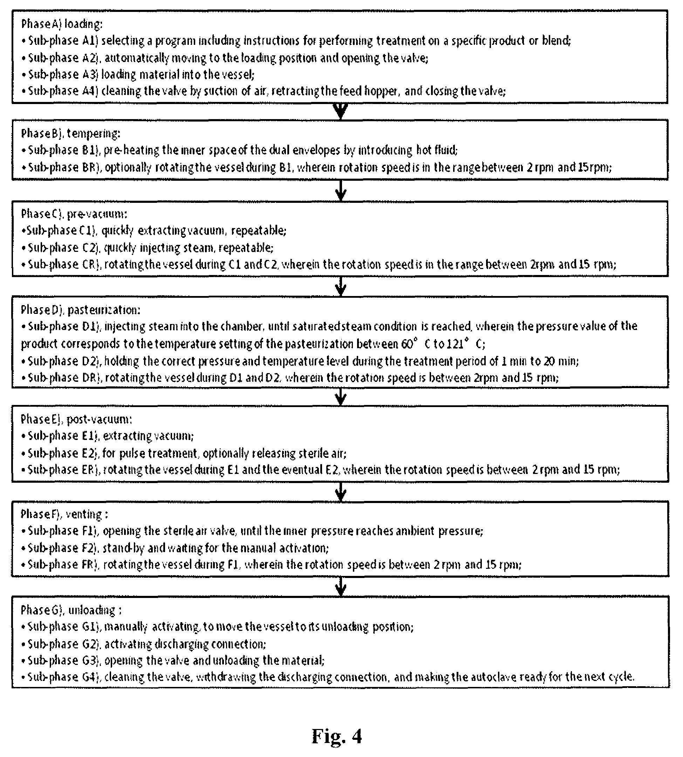

Said rotary autoclave 10, constructed as described above, allows intended objects on industrial scale to be achieved by means of a particulate automatic treatment process in the type of continuous operation phases (phases A-G), including the respective sub-phases (sub-phases A1 - G4), the automatic treatment process is the process described below and also summarized in the simplified schematic diagram (Fig. 4).

The phase A, otherwise called loading phase of material or materials (if a plurality of types), includes at least the following sub-phases:
- Sub-phase A1) the process is initialized by selecting a specific program, for example, by means of a conventional touch screen connected to said PLC type electronic controller163, wherein the specific program activates sequentially said phases and sub-phases by properly setting the values for the main parameters at least characterizing following treatments: such as, the temperature of the granular product, the temperature of the treatment chamber, the temperature of the double jacket, the pressure, the duration of the pasteurization phase, the rotation speed; therefore, said program substantially corresponds to the collection of standardized information and settings which, depending on the particular product and the treatment to be performed, automatically and accurately adjusts the rotary autoclave 10. Additionally, through said PLC type electronic controller, there are at least standardized recordings such as, cycle number, lot number, description of the treated product, and all the treatment parameters which are critical for the effective pasteurization of the product, like temperature, pressure and time values.
- Sub-phase A2) The rotary autoclave 10 is rotated to the position for loading the granular product 100 with a highest point 110a of the loading position of the inclined vessel, the highest point 115a of the main opening loading position is positioned on the highest point 151 a (Fig. 1) of the top loading position directly underneath the external feeding hopper 170, which preferably is of a type of discharge by gravity, or by pressure, and actively opens the valve 116 integrally incorporated in the highest point 115a of the main opening loading position;
- Sub-phase A3) the external feeding hopper 170 is engaged and then the material to be treated, also called batch, is discharged. The batch may consist of one or several different products which, generally but not exclusively, are loaded sequentially;
- Sub-phase A4) once the loading is finished, the cleaning of the opening 115 and the valve 116 is performed by means of suction of air, so as to eliminate the residual material, and then the external feeding hopper 170 is withdrawn and the valve is closed.

The phase B), otherwise called tempering, is designed to preheat the granular product 100 to a certain temperature level and for a certain duration according to the program, in addition, if required by the type of product 100, the blend can be constituted by a single kind of material or an aggregate of materials, in order to standardize and accelerate the processing, said phase B comprises at least the following sub-phases:
- Sub-phase B1) automatically preheating the treatment cavity 113 of the double jacket (the first jacket 111, the second jacket 112) by means of a hot fluid such as steam or water, according to the program mentioned in sub-phase Al, the product 100 being heated through convection by the inner wall of the second jacket 112;
- Sub-phase B2) automatically rotating the inclined vessel 110 around the horizontal rotation axis 150, according to the program mentioned in the sub-phase A1, which causes the product 100 to be blended in a particularly efficient manner, for the purpose of homogeneously heating the product due to the particular inclined configuration (the longitudinal axis 151a, and the longitudinal axis 151 b) and the limited rotation speed which could be set between 2 rpm and 15rpm. In the preferred embodiment, said sub-phases B1 and B2 are performed simultaneously and during the complete time period set in the program mentioned in the sub-phase A1.

The phase C), otherwise called pre-vacuum, allows raising the temperature of the product 100 in the treatment chamber 114 in a short time by means of rapid vacuum extracting by the first vacuum pump 131 and the second vacuum pump 131 and the following steam injection by the first steam generator 133 and the second steam generator 134. Both said vacuum extraction and said steam injection are performed exclusively through the single filter (the second coupling 130, the first filtration system 137), the second coupling 130 of the filter and the first filtration system 137 are called wedge-shaped wire screen of bidirectional and self-cleaning type (Figs. 1 and 3). In this phase, the vessel 110 is rotated at a set speed set by the program in such a way as to facilitate homogeneously removing the air and optimizing the penetration of the steam through the entire volume of the material. Said phase C includes, therefore, at least the following sub-phases:
- Sub-phase C1) fast and automatically withdrawing air from the treatment chamber 114 by establishing vacuum according to a target pressure value set in the program mentioned in the sub-phase A1;
- Sub-phase C2) automatically injecting the steam into the treatment chamber 114, until the target pressure value set in the program mentioned in the sub-phase A1 is reached;
- Sub-phase CR) automatically rotating the vessel 110 according to the program mentioned in the sub-phase A1 during the sub-phase C1 and the sub-phase C2.

In particular, said sub-phases C1 and C2 can be repeated several times, if required by said program. In this regard, it is noted that the phase C always begins with the vacuum extraction referred to as the sub-phase C1, and ends with the steam injection referred to as sub-phase C2, in order to optimize the steam penetration and to properly condition the product 100 in the subsequent phase D. Depending on the specific material to be treated and the specific treatment to be performed, therefore, said step C is performed once or may be repeated aiming to the proper penetration of steam into the whole volume of product. For both the vacuum phase and the injection of steam, this phase C is controlled to reach the target pressure value set by the program, with the objective that this phase C is ended successfully in the shortest possible time so that the product is heated with a minimum pick up of moisture.

The phase D), generally called pasteurization phase, is a main phase for the purpose of reducing the bacterial load, and provides that, starting from the conditions obtained from the previous phase C, steam is automatically injected into the treatment chamber 114 until the pressure corresponds to the saturated steam condition, and therefore, when the product 100 reaches the temperature of pasteurization as defined by said program mentioned in the sub-phase A1, it starts to measure the actual duration of the treatment. Then adjustment of the variable parameters during this phase D is done according to the known properties of the saturated steam, i.e. by setting the values of pressure in the treatment chamber 114, which corresponds to the particular temperature value of the product 100, it is also possible to adjust the temperature of the treatment cavity 113 of the double jacket, the fluid in the external heater 121, the first conditioning fluid 122 and the second conditioning fluid 123. Said phase D includes at least the following sub-phases:
- Sub-phase D1) automatically injecting the steam into the treatment chamber 114 until the temperature in the product 100 reaches the correct temperature level for its pasteurization or sterilization;
- Sub-phase D2) automatically maintaining the correct level of pressure in the treatment chamber 114 and the pasteurization temperature in the material product 100 for the whole duration of the heat treatment;
- Sub-phase DR) automatically rotating the vessel 110 according to the values set in the program mentioned in the sub-phase A1 during the sub-phase D1 and the sub-phase D2.

In particular, the invention provides that the correct level of pasteurization temperature, as described above, is between 60°C and 121°C, while the correct duration of this heat treatment is typically but not limited to between 1 min. and 20 min., these parameters are set and automatically controlled in the procedure A1, depending on the specific material or combination of materials to be treated and the envisaged specific treatment. As an example, but not limited to it, the treatment values defined for a conventional pasteurization treatment of a mixture of oatmeal with raisins and walnut pieces are a pasteurization temperature of 95°C for a duration of at least 8 min.; furthermore, for a conventional sterilization treatment for vanilla powder, the defined level of temperature is 115°C within a duration of at least 12 min.

It is also noted that, since the integrated control system is mainly consisted of the chamber temperature probe 160, the product temperature probe 161, the pressure transducer 162 and the electronic controller 163 that automatically adjusts the correct levels of pressure with the variation of the set temperature, said rotary autoclave 10 is able to accurately maintain the interior of the treatment chamber 114 in the saturated steam conditions for a long time, it is also done by means of a modulating type valve for controlling the amount of the steam injected in the chamber, and for this purpose, the steam injection through the first filtration system 137 and the temperature of the treatment cavity 113 between the double jackets can be continuously adjusted. Additionally, it is noted that said autoclave allows creating and maintaining such conditions in an optimized way even during the blending action, and is designed to rotate independently with respect to the various accessory devices for controlling the inner condition of the first coupling 120, the external heater 121, the first conditioning fluid 122, the second conditioning fluid 123, the second coupling 130, the first vacuum pump 131, the second vacuum pump 132, the first steam generator 133, the second steam generator 134, the first filtration system 137 of the chamber. The autoclave is rotated at a speed set automatically by the program mentioned in the sub-phase A1 in such a way as to optimize the exposure of the product to said saturated steam in a homogeneous manner; and when the set time for the pasteurization or sterilization treatment lapses, it automatically switches to the subsequent phase E.

The phase E), also called post-vacuum phase, is designed to cool and dry the material product 100 for a duration which is dependent on the product to be treated and defined by the program mentioned in the sub-phase A1. Said phase E includes, therefore, at least the following sub-phases:
- Sub-phase E1) automatically creating vacuum inside the treatment chamber 114 according to the pressure target value defined in the program mentioned in the sub-phase A1.
- Sub-phase E2) optionally, in a pulsated drying configuration, releasing ambient air through an external sterile filter by opening a valve, until a pre-selected target pressure has been reached.
- Sub-phase ER) automatically rotating the vessel 110 according to the program mentioned in the sub-phase A1, while rotating during the sub-phase E1 and the eventual sub-phase E2 aims to homogenize the treatment.

In particular, for said phase E, there is an option to choose between a pulse type post-vacuum where vacuum cycles as mentioned in the sub-phase E1 are alternating with the injection of sterile air as mentioned in the sub-phase E2 for a pre-selected duration, and a fixed type simple post-vacuum where the minimum pressure target has to be reached as mentioned in the sub-phase E1 and then is maintained during a predefined time. The vacuum extraction is done through the first filtration system 137 in the form of the wedge-shaped wire screen to avoid that powders are made by the autoclave 110 through the first vacuum pump 131. When the ambient air is introduced into the autoclave, this ambient air will pass through the external sterile filter to avoid any contamination of the product by the air. Due to the pressure differential between the negative pressure inside the autoclave and ambient pressure outside, the air will naturally flow into the autoclave. Once the defined post-vacuum time is reached, the system will automatically switch to the subsequent phase F.

The phase F), otherwise called venting phase, is suitable to return the treatment chamber 114 to atmospheric pressure in order to perform the unloading of the product. This phase F, therefore, includes at least the following sub-phases:
- Sub-phase F1): automatically opening the valve connected to the external sterile filter, naturally releasing sterile air into the treatment chamber 114 still under vacuum conditions;
- Sub-phase F2): reaching the ambient pressure level, for safety reasons, the autoclave remains stand-by, waiting for the manual activation of the next and final phase G;
- Sub-phase FR) automatically rotating the vessel 110 according to the program mentioned in the sub-phase A1 during the sub-phase F1.

The phase G), otherwise referred to as discharge phase, for safety reasons, is manually activated by the operator, preferably by means of the touch screen connected with the electronic controller 163, to initiate the following sub-phases:
- Sub-phase G1), automatically rotating the autoclave 10 to its unloading position with a lowest point 110b (Fig. 1), so that the lowest point 115b of main opening at the unloading position is positioned at the bottom of an external discharging recipient;
- Sub-phase G2), activating said connection, which can alternatively directly discharge by gravity into a hopper placed below the lowest point 115b of the main opening at the unloading position or into a dense phase pneumatic transport line according to the requirement of the client;
- Sub-phase G3) opening the valve (116) integrated in the lowest point 115b of the opening at the unloading position and performing the complete discharge of the treated product 100;
- Sub-phase G4) finishing the discharging, wherein said valve 116 is cleaned by an air suction system for the residual material, and subsequently the autoclave 10 is ready for the next cycle according to said automatic treatment process.

Consequently, the organic bulk material with a low moisture content in powder form or as particulates, which is loaded into the treatment chamber 114 of said rotary autoclave 10 can be industrially processed according to the automatic treatment process described above, the above described problems can be advantageously solved, and the intended object is achieved. Furthermore it is noted that, thanks to the particular constructive configuration of said rotary autoclave 10, it is possible to perform combined blending and pasteurization - sterilization treatments for any organic bulk material with a low moisture level in the form of powder or as particulates, the process is customizable with respect to a wide variability of all operational parameters characterizing said treatment system, especially for pressure, temperature and uniformity of application, and also according to new programs for new products. Additionally, the invention allows to optimize the cleaning to significantly reduce the possibility of contamination, and for this purpose, for example, a particular bidirectional self-cleaning first filter 137 is provided, and also it is expected that when materials are changed, an automatic cleaning cycle is performed, which can be reinforced by enhanced washing through a manhole 117 type opening which allows periodically entering inside the treatment chamber 114 for a complete and easy cleaning of all surfaces that are in contact with the material, wherein all the surfaces are particularly smooth and have no internal moving elements or protruding parts.

The invention described above also enables the material product 100 to be advantageously treated in an automatic way, to achieve a high quality at a reduced operating cost, and also to be suitable for high yield.

By way of a non-limiting example, an example of a practical implementation of the rotary autoclave 10 and the automatic treatment process is provided below, which is related in particular to a pasteurization treatment optimized for a mixture of cereals including oatmeal, raisins and pieces of walnuts. For this purpose, said automatic treatment process 20 provides the phases and sub-phases defined as follows:
Phase A), loading:
   - Sub-phase A1), selecting treatment program on the touch screen;
   - Sub-phase A2), moving to the loading position and opening the valve, identical for all products;
   - Sub-phase A3), standard loading/starting procedure, identical for all products;
   - Sub-phase A4), cleaning and closing the valve, retracting the feed hopper, identical for all products;
Phase B), tempering:
   - Sub-phase B1), pre-heating to the target product temperature: 30 °C
   - Sub-phase BR), rotation speed: 8 rpm, during the sub-phase B1;
Phase C), pre-vacuum:
   - Sub-phase C1), initiating the pre-vacuum with target level of -0.910 bar;
   - Sub-phase C2), injecting steam at the pressure target level of 1 bar;
   - Sub-phase CR), rotation speed: 8 rpm, during the sub-phases C1 and C2;
• Phase D), pasteurization:
   - Sub-phase D1), stopping injecting the steam at target pressure of 1 bar to reach the specified pasteurization temperature of 100°C;
   - Sub-phase D2), regulating the temperature of the double jacket and the pressure of the chamber to maintain the saturated steam condition during the specified pasteurization time of 9 minutes;
   - Sub-phase DR), rotation speed: 8 rpm, during the sub-phases D1 and D2;
Phase E), post-vacuum:
   - Sub-phase E1), extracting vacuum by opening the vacuum valve until the set target pressure of -0.820 bar has been reached;
   - Sub-phase E2), continue performing vacuum extraction during a set target time of 10 minutes;
   - Sub-phase ER), rotation speed: 8 rpm, during the sub-phases E1 and E2;
Phase F), venting:
   - Sub-phase F1), introducing the ambient air through a sterile filter into the autoclave until the pressure inside the autoclave reaches ambient pressure;
   - Sub-phase F2), stand-by, waiting for the manual activation;
   - Sub-phase FR), rotation speed: 8 rpm, during the sub-phase F1;
Phase G), unloading:
   - Sub-phases G1 - G4), standard discharging procedure, identical for all products.

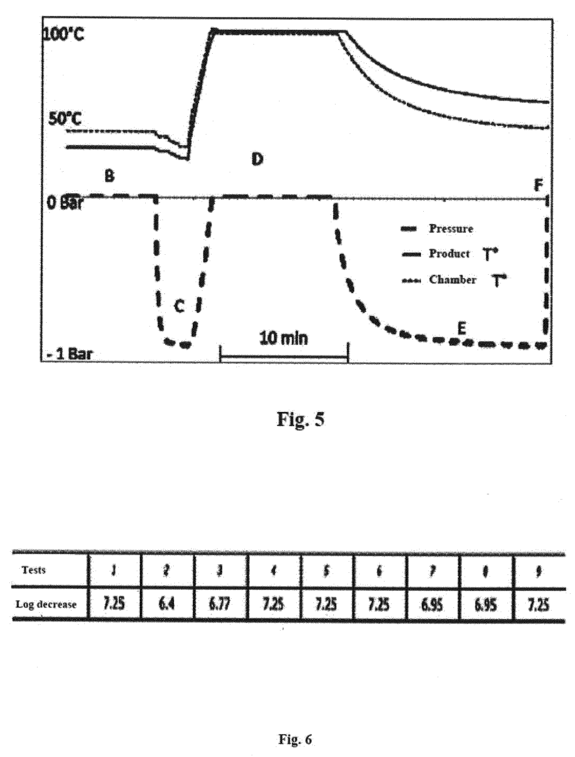

With the rotary autoclave (10) realized as described by the present invention and used according to said automatic treatment process, it is possible to achieve an improved pasteurization and blending performance, compared to conventional solutions. By way of non-limiting example, as shown graphically (Fig. 5), the parameters of the pasteurization cycle optimized for the pasteurization of a mixture of cereals including oatmeal, raisins and pieces of walnuts is depicted as a double diagram, which are in direct relation with the values of temperature and pressure during said phases B - F. In particular, it was found that such a process of pasteurization is extremely effective and also repeatable; in more detail, a series of nine cycles of pasteurization has been performed on said cereal mixture which has been mixed with enterococcus faecium, and the effectiveness of said treatment was measured in terms of Log decrease (Fig. 6) on a logarithmic scale, to define the value of the eliminated pathogenic microbes, which, as a result, is comprised between a minimum of 6.4 and a maximum of 7.25 and average of which equals to about 7, which corresponds to about as many as one hundred times the decrease of the bacterial load, compared to the values that are usually achieved with conventional solutions of pasteurization used today, and which corresponds to approximately 5 Log decrease.

Additionally, it is noted that the parameter that measures the quality of mixing and which is usually referred to as blending performance is excellent with respect to said mixture of cereals including oatmeal, raisins and pieces of walnuts and particularly treated as described above (Fig. 5), and so are the moisture values, measured as percentage in the samples taken during the phase G of unloading and during the phase A of loading.

### Note:

The rotary autoclave 10 is a rotary autoclave for use under the saturated steam condition and considered as a complex and automatic equipment, and incorporates integrally a plurality of members, functions and its combination as described in the present invention.

The product 100 is a product or a mixture of the product to be treated, the vessel 110 is a rotary vessel has a inclined axis, the highest point 110a of the loading position is shown at the vessel at the loading position, and the lowest point 110b of the unloading position is shown at the vessel at the unloading position, the first jacket 111 constructs an outer wall, the second jacket 112 constructs an inner wall, the treatment cavity 113 is a cavity of which the temperature of the double jacket is controlled, the treatment chamber 114 is a internal treatment chamber, and the surface 114a is an inner surface of the treatment chamber, the opening 115 is an opening for loading and unloading, and the highest point 115a of the loading position is shown at the opening at the loading position, and the lowest point 115b of the unloading position is shown at the opening at the unloading position, and the support legs 119 are support legs for the vessel.

The first coupling 120 is a double channel rotary coupling, the external heater 121 is an external heater having a fluid circulation system, the first conditioning fluid 122 is flowed in from an inlet for hot fluid, and the second conditioning fluid 123 is a return flow of the fluid. The flange 124 is a flange to be connected with the vessel, the outer pipe 126 is a fluid return pipe, the second coupling 130 is a single channel fixed coupling, the first vacuum pump 131 is an external vacuum pump, the second vacuum pump 132 is used to generate vacuum extraction flow, the first steam generator 133 is an external steam generator, the second steam generator 134 is used to generate a steam injection flow, and the first filtration system 137 is a fixed filter of the wedge-shaped wire screen type.

The motor 140 includes a gearbox, the chain system 141 includes a transmission chain, the rotary support 144 is a fixed support for rotation, the sensor 145 is a fixed sensor for positioning, the rotary locator 146 is a rotary reference member, and the collector 147 is a collector for data transmission.

The longitudinal axis 151 is the longitudinal axis of the vessel, the longitudinal axis 151a is the longitudinal axis at the loading position, and the longitudinal axis 151 b is the longitudinal axis at the unloading position.

The particular embodiment of the present invention has been described above. It is appreciated that the present invention is not limited to the above particular embodiment, and variations and modifications can be made within the scope of the claims by the person skilled in the art, without influencing the substantive content of the present invention.

## Claims

1. An automatic rotary autoclave (10), which is used under saturated steam condition for pasteurization - sterilization, blending and mixing of a low moisture product (100) in particulate form, comprising: a hermetically closed cylindrical vessel (110) having a complete treatment chamber (114) and of a double jacket (111, 112) type, the double jacket (111, 112) having a treatment cavity (113), and the vessel (110) being moved by an electrical motor, **characterized in that** the vessel (110) has a longitudinal axis (151) inclined to a horizontal rotation axis (150), wherein the treatment chamber (114) of the vessel (110) has a inner surface (114a) which is smooth and has no protrusions, or a member for mechanically blending the product (100), and wherein the vessel (110) at least has:
means (140 ∼ 144, 150) for supporting and rotating the vessel;
means (121 ∼ 123) for controlling temperature of said treatment cavity (113) of the double jacket through a first coupling (120) as a rotary coupling positioned to be connected with said horizontal rotation axis (150);
means (131 ∼ 134, 137) for supplying said treatment chamber (114) alternately with steam injection or vacuum extraction through a second coupling (130) as a fixed coupling, wherein on the opposite sides of the means (121 ∼ 123) for controlling temperature with respect to said treatment cavity (113), the vessel also has a system (137, 138) for filtration and diffusion inside the treatment chamber (114), said second coupling (130) as the fixed coupling is positioned to be connected with said horizontal rotation axis (150), and the treatment chamber (114) is connected with the second coupling (130) as the fixed coupling and is of a self-cleaning type;
sensor means (145, 150) for detecting rotation and positioning of the vessel (110) with respect to the horizontal rotation axis;
a PLC type electronic controller (163) which is connected in an integrated way at least to said means (121 ∼ 123) for controlling temperature, said means (131 ∼ 134) for supplying, and said means (140 ∼ 144) for supporting and rotating, and at least connected to temperature and pressure sensing means (160 ∼ 162) and a rotation sensing means (145) in order to monitor and automatically adjust values of pressure and temperature in the treatment chamber (114) so as to achieve and maintain the correct treatment conditions of the product (100) even during the rotation of the vessel (110) and the presence of the saturated steam, said electronic controller (163) being adapted to manage automatically the operation of the autoclave (10) and the whole treatment process.

2. The automatic rotary autoclave (10) according to claim 1, **characterized in that** said vessel (110) is rotated on the horizontal rotation axis (150) in such a way that the vessel (110) can reach its loading position at which the opening (115) is located at the highest point (110a, 115a, 151a) of the vessel or its unloading position at which the opening (115) is located at the lowest point (110b, 115b, 151b) of the vessel, said opening (115) is also equipped with a valve (116); said vessel (110) is used as an agitator-blender combining low speed rotation with the gravitational mass of the product (100); and said means (121 ∼ 123) for controlling the temperature of the treatment cavity (113) comprise an external heater (121) with an agitator for hot fluid (122, 123) which is introduced through the first coupling (120) as a rotary coupling, the first coupling (120), as a rotary coupling, is of the dual channel concentric type, and wherein said means (131 ∼ 134) for supporting the treatment chamber (114) comprise at least one vacuum pump (131 ∼ 132) and a steam generator (133, 134), said second coupling (130) as a fixed coupling is of a single-channel and bi-directional type and also provides a mechanical seal (136) of the vessel (110) with a closure member in such a way as to allow the vessel (110) to be rotated simultaneously and independently during the vacuum extraction and during the steam injection, and wherein said filtration and diffusion system is achieved by filters (137, 138) of the wedge-shaped wire screen type, and wherein said temperature and pressure sensing means (160 ∼ 162) combines the sensors for detecting the pressure and temperature of the hot fluid (122, 123) in the treatment cavity (113) of the double jacket, and wherein said electronic controller (163) is electronically integrated with said valve (116), said means (121 ∼ 123) for controlling temperature, said means (131 ∼ 134) for supplying, said means (140 ∼ 142) for rotating, said temperature and pressure sensing means (160 ∼ 162) in such a way as to automatically manage the operation of the vessel according to an automatic treatment method which is selected when the cycle is initiated according to the product (100) to be treated and the treatment to be performed; said treatment method enables to instruct said controller (163) with the correct operating parameters in such a way that the correct saturated steam conditions are achieved and maintained in the whole treatment chamber (114) during the rotation of the vessel.

3. The automatic rotary autoclave (10) according to claim 1 or 2, **characterized in that** said vessel (110) is made of food grade stainless steel metal, and has an inner capacity of the treatment chamber (114) in the range between 200 and 30000 liters; and wherein said vessel (110) has said longitudinal axis (151) inclined with respect to the horizontal rotation axis (150) of the autoclave (10) by an angle in the range between 30° and 40°; and wherein said speed of slow rotation is in the range between 2 rpm and 15rpm.

4. A. treatment process having continuous operation phases by means of the automatic rotary autoclave as claimed in claim 1, wherein said process at least provides, in a logic sequence, the following phases A ∼ G:
Phase A), loading phase, including at least: sub-phase A1) initiating a predefined program that is from the electronic controller and standard recorded; sub-phase A2): entering a loading position through the opening of the valve; sub-phase A3) loading the product in the vessel; sub-phase A4) cleaning the valve by air suction, retracting an external feeding hopper and closing the valve;
Phase B), tempering phase, allowing the preheating of the material, including at least: sub-phase B1) heating the treatment cavity by introducing hot fluid, steam or water;
Phase C), pre-vacuum phase, increasing the temperature of the treatment chamber in a short time up to a predetermined pressure level by means of a rapid vacuum extraction followed by a rapid steam injection, and wherein the vacuum and the steam pass through the same single bi-directional and self-cleaning filter, and wherein the phase C) is performed once or in a repetitive manner, always beginning with the vacuum and terminating at steam injection, wherein said phase C includes at least: sub-phase C1): achieving a rapid vacuum; sub-phase C2): rapidly injecting steam; sub-phase CR): slowly rotating the vessel (110) during the sub-phase C1 and the sub-phase C2;
Phase D), pasteurization phase, wherein in order to maintain the correct pasteurization or sterilization temperature under saturated steam conditions, adjustment is provided by the controlled injection of steam into the chamber (114) and the temperature control of the treatment cavity (113), said phase D includes at least: sub-phase D1): injecting steam into the treatment chamber (114), wherein pressure values on the product (100) are achieved corresponding to the pre-selected temperature, which is in the range between 60°C and 121°C; sub-phase D2) maintaining the pressure and temperature levels during the whole defined duration of the treatment; and sub-phase DR) slowly rotating the vessel (110) during the sub-phase D1 and the sub-phase D2;
Phase E), post-vacuum phase, cooling and drying the material, including at least: sub-phase E1) extraction of vacuum; sub-phase E2) eventually, in pulse type treatment, releasing air through a valve with a sterile air filter; and sub-phase ER) slowly rotating the vessel (110) during the sub-phase E1 and the eventual sub-phase E2;
Phase F), venting phase, allowing the treatment chamber to return to atmospheric pressure for discharging, including at least: sub-phase F1) opening the valve to release sterile air into the treatment chamber until reaching the ambient pressure; sub-phase F2) stand-by and waiting for manual activation; and sub-phase FR) slowly rotating the vessel (110) during the sub-phase F1;
Phase G), unloading phase, manually activating, including at least: sub-phase G1) moving the vessel (110) to the unloading position; sub-phase G2) activating the discharge connection; sub-phase G3) opening the valve and unloading the material product; sub-phase G4) cleaning the valve, and making the autoclave (10) ready for the subsequent processing cycle.

5. The treatment process having continuous operation phases by means of the automatic rotary autoclave as claimed in claim 1 according to claim 4, **characterized in that** in the sub-phase D2), the pressure and the temperature level during the whole defined duration of the treatment is maintained for a duration in the range between 1 min. and 20 min,

6. The treatment process having continuous operation phases by means of the automatic rotary autoclave as claimed in claim 1 according to claim 4, **characterized in that** the phase B) includes sub-phase BR) of slowly rotating the vessel (110) during the sub-phase B1.
